(19) Europäisches Patentamt
European Patent Office
Office européen des brevets

(11) Veröffentlichungsnummer: **0 004 098**
**B1**

(12) # EUROPÄISCHE PATENTSCHRIFT

(45) Veröffentlichungstag der Patentschrift: **12.05.82**

(51) Int. Cl.³: **C 07 J 9/00,** C 07 J 53/00 // C07D317/16

(21) Anmeldenummer: **79100748.7**

(22) Anmeldetag: **13.03.79**

(54) **Cholestanderivate und Verfahren zu deren Herstellung.**

(30) Priorität: **15.03.78 CH 2818/78**

(43) Veröffentlichungstag der Anmeldung: **19.09.79 Patentblatt 79/19**

(45) Bekanntmachung des Hinweises auf die Patenterteilung: **12.05.82 Patentblatt 82/19**

(84) Benannte Vertragsstaaten: **BE CH DE FR GB IT NL**

(56) Entgegenhaltungen:
**US-A-3 928 397**
**JOURNAL OF THE CHEMICAL SOCIETY PERKIN TRANSACTIONS I, 1976, Band 7 London, GB, STEPHEN C. EYLEY et al.: »Synthesis of 25-Hydroxyprovitamin $D_3$ and 25,26-Dihydroxy provitamine $D_3$«, Seiten 731-735**

(73) Patentinhaber: **F. HOFFMANN-LA ROCHE & CO. Aktiengesellschaft, CH-4002 Basel (CH)**

(72) Erfinder: **Barner, Richard, Dr., In den Reben 97, CH-4108 Witterswil (CH)**
Erfinder: **Hübscher, Joseph, Spausel 1080, CH-5703 Seon (CH)**

(74) Vertreter: **Lederer, Franz, Dr. et al, Patentanwälte Lederer, Meyer Lucile-Grahn-Strasse 22, D-8000 München 80 (DE)**

**EP 0 004 098 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Cholestanderivate und Verfahren zu deren Herstellung

Die vorliegende Erfindung betrifft neue Cholestanderivate, die als Zwischenprodukte für die Herstellung von 25,26-Dihydroxy-cholecalciferol Verwendung finden können, sowie deren Herstellung.

Hydroxyderivate von Vitamin D beanspruchen seit einiger Zeit besonderes Interesse, da eine Reihe solcher Derivate als biologisch wirksame Metabolite aufgefunden wurden. Zu diesem Metaboliten zählt das 25,26-Dihydroxy-cholecalciferol (Biochemistry 9, 4776, 1970). Da diese Stoffe aus natürlichen Quellen in größeren Mengen nicht verfügbar sind; kommt ihrer Synthese besondere Bedeutung zu. Für das 25,26-Dihydroxy-cholecalciferol sind mehrere Synthesen konzipiert worden (Compt. rend. 285, serie D, 443 (1977)). Diese Synthesen führen zu Epimerengemischen von 25R- und 25S-Verbindungen, deren Trennung sich schwierig gestaltet. Mit der vorliegenden Erfindung wird ein neues Verfahren zur Verfügung gestellt, das es ermöglicht, das 25R,26- und das 25S,26-Dihydroxy-cholecalciferol nun auch stereoselektiv herzustellen.

Ein Aspekt der vorliegenden Erfindung betrifft neue Cholestanderivate der Formel

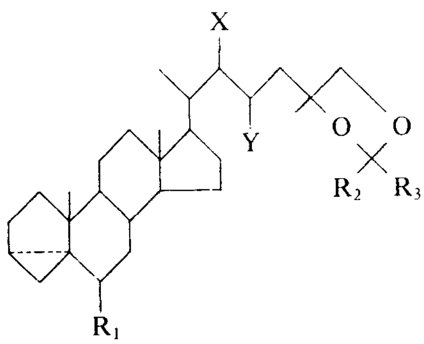

(Ia)

worin $R_1$ nieder-Alkoxy, $R_2$ und $R_3$ nieder-Alkyl oder $R_2$ und $R_3$ zusammen nieder-Alkylen, X Hydroxy, eine Gruppe der Formel $OSO_2R_4$ oder Wasserstoff und Y Wasserstoff oder X und Y zusammen eine C-C-Bindung und $R_4$ nieder-Alkyl, Phenyl oder substituiertes Phenyl sind,

insbesondere Cholestanderivate der Formel

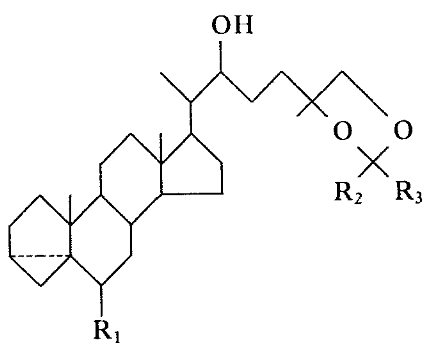

(I)

worin $R_1$, $R_2$ und $R_3$ die obige Bedeutung haben.

Ein weiterer Aspekt der vorliegenden Erfindung betrifft ein Verfahren zur Herstellung von 25,26-Dihydroxycholesterin, das dadurch gekennzeichnet ist, daß man

a) einen i-Steroidaldehyd der Formel

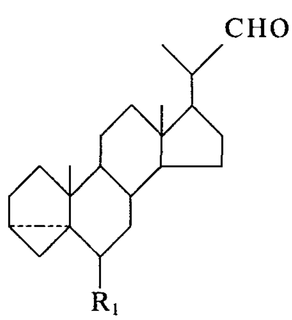

(II)

worin $R_1$ nieder-Alkoxy ist,
mit einem Halogenketal der Formel

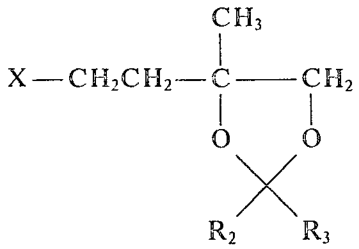

(III)

worin X Chlor, Brom oder Jod ist und $R_2$ und $R_3$ nieder-Alkyl oder $R_2$ und $R_3$ zusammen nieder-Alkylen sind,
in einer Grignard-Reaktion umsetzt,
b) den erhaltenen 22-Steroidalkohol der Formel

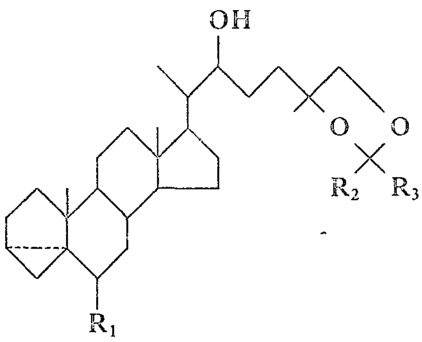

(I)

worin $R_1$, $R_2$ und $R_3$ die obige Bedeutung haben,
mit einer Sulfonsäure verestert,
c) den erhaltenen 22-Sulfonsäureester der Formel

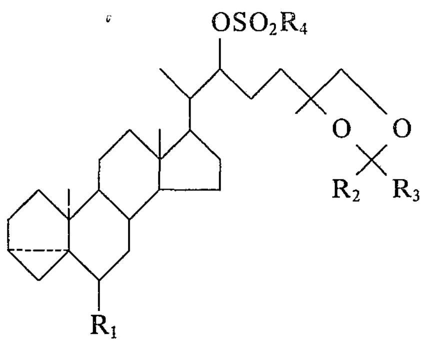

(IV)

worin $R_1$, $R_2$ und $R_3$ die obige Bedeutung haben und $R_4$ nieder-Alkyl, Phenyl oder substituiertes Phenyl ist,
entweder direkt durch Behandlung mit einem komplexen Metallhydrid oder zweistufig durch Abspaltung der Sulfonsäure und unter Ausbildung einer 22,23-Doppelbindung und Hydrierung des erhaltenen Steroidketals der Formel

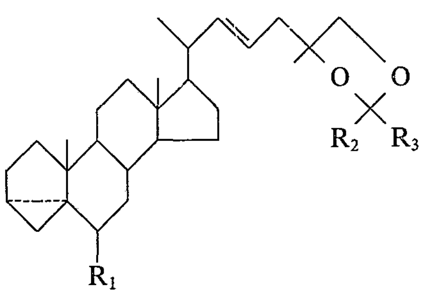

(VI)

worin $R_1$, $R_2$ und $R_3$ die obige Bedeutung haben, in ein Steroidketal der Formel

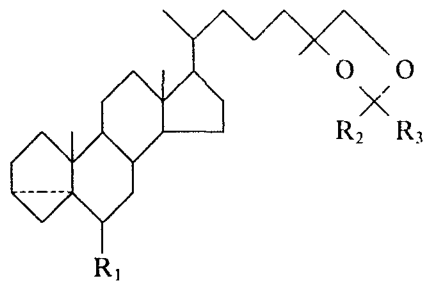

(V)

worin $R_1$, $R_2$ und $R_3$ die obige Bedeutung haben, überführt,

d) das Steroidketal der Formel V einer retro-i-Umlagerung unterwirft und die Ketalgruppe hydrolysiert.

Es wurde bereits vorgeschlagen (J. C. S. Perkins Trans. I, 1976, Band 7, 731), die 25,26-Dihydroxycholesterinseitenkette in ein tetracyclisches Steroidgerüst durch Grignard-Reaktion eines 21-Steroidaldehydes mit dem entsprechenden Grignard-Reagens einzuführen. Dieser Versuch scheiterte an der angeblich mangelenden Reaktivität des Halogenids gegenüber Magnesium und Lithium. Somit bestand ein Vorurteil gegen die Herstellbarkeit des Grignard-Reagens bzw. die Herstellbarkeit von 25,26-Dihydroxycholesterin auf dem zur Diskussion stehenden Weg. Dieses Vorurteil wurde nun überwunden, indem das Grignard-Reagens hergestellt werden konnte und aus letzterem die für das 25,26-Dihydroxycholesterin charakteristische Seitenkette in das 21-Steroidaldehyd der Formel II eingeführt werden konnte.

Die Grignard-Reaktion zwischen dem i-Steroidaldehyd der Formel II und dem Halogenketal der Formel III kann in einem für Grignard-Reaktionen üblichen Lösungsmittel, z. B. einem Äther, wie Tetrahydrofuran, durchgeführt werden. Die Reaktionstemperatur wird zweckmäßig zwischen 0° C und Raumtemperatur gehalten. Vorzugsweise verwendet man eine Verbindung der Formel III, in der X Brom ist. Beispiele von niederen Alkylresten $R_2$ und $R_3$ sind Methyl, Äthyl und Propyl, wobei Methyl bevorzugt ist. Beispiele für durch $R_2$ und $R_3$ gemeinsam gebildete Alkylenreste sind Äthylen und Propylen. Beispiele von niederen Alkoxygruppen $R_1$ sind $C_{1-6}$-Alkoxyreste, insbesondere Methoxy.

Das Reaktionsprodukt der Grignard-Reaktion kann in üblicher Weise, z. B. durch Hydrolyse mit schwachen Säuren, wie wäßrige Ammoniumchloridlösung, aufgearbeitet werden.

In einer bevorzugten Ausführungsform dieses Verfahrens setzt man einen (20S)-i-Steroidaldehyd der Formel II mit einer optisch aktiven Verbindung der Formel III bzw. deren Grignard-Verbindung, insbesondere dem (4S)-4-(2-Bromäthyl)-2,2,4-trimethyl-1,3-dioxolan um. Bei Verwendung einer optisch aktiven Verbindung der Formel III erhält man eine Verbindung der Formel I mit 25R- oder 25S-Konfiguration, je nachdem, welche Chiralität die Verbindung der Formel III aufweist.

Die Veresterung der 22-Hydroxygruppe in einem Steroid der Formel I kann durch Umsetzung mit einem reaktiven Sulfonsäurederivat der Formel $R_4SO_2Y$ worin Y Brom, Chlor oder $R_4SO_2O$- darstellt und $R_4$ die obige Bedeutung hat, in einem geeigneten organischen Lösungsmittel, vorzugsweise in Gegenwart eines Säureakzeptors durchgeführt werden. Beispiele von Alkylsulfonsäuren sind Methan- und Äthansulfonsäure, Beispiele von substituierten Benzolsulfonsäuren sind nieder-Alkylphenylsulfonsäuren wie p-Toluolsulfonsäure, oder p-Nitrobenzolsulfonsäure. Geeignete organische Lösungsmittel für die Veresterung sind aromatische Lösungsmittel wie Benzol, Toluol und Xylol oder hetero-aromatische Lösungsmittel wie Pyridin, Picolin oder Collidin. Als Säureakzeptoren kommen beispielsweise aliphatische Amine wie Triäthylamin, oder aromatische Amine wie Dimethylanilin oder hetero-aromatische Amine wie Pyridin, Picolin oder Collidin in Betracht. In letzterem Fall kann der Säureakzeptor gleichzeitig als Lösungsmittel dienen.

Der Sulfonylester IV kann durch Behandlung mit einem komplexen Metallhydrid, wie Lithiumaluminiumhydrid, in eine Verbindung der Formel V übergeführt werden. Diese reduktive Abspaltung der Sulfonsäureestergruppe kann in einem inerten organischen Lösungsmittel z. B. bis zur Rückflußtemperatur des Lösungsmittels, durchgeführt werden. Bei dieser Reaktion kann als Nebenprodukt eine Verbindung der Formel VI erhalten werden, die durch katalytische Hydrierung in eine Verbindung der Formel V umgewandelt werden kann.

Alternativ kann der Sulfonylester IV in eine Verbindung der Formel VI übergeführt werden, die wiederum durch Hydrierung in eine Verbindung der Formel V umgewandelt werden kann. Die Abspaltung der Sulfonylestergruppe aus einer Verbindung der Formel IV unter Bildung einer Verbindung der Formel VI kann durch Behandlung mit Basen, wie N,N-Dimethylanilin, Pyridin, Collidin, oder mit Lithiumbromid oder Lithiumcarbonat, oder einem Gemisch von Lithiumbromid und Lithiumcarbonat durchgeführt werden. Die Hydrierung einer Verbindung der Formel VI zu einer

Verbindung der Formel V kann katalytisch, z. B. in Gegenwart von Edelmetallen wie Platin, Palladium oder Rhodium, oder mit Nickelkatalysatoren durchgeführt werden.

Die retro-i-Umlagerung eines Steroids der Formel V kann durch Behandlung mit einer Säure in einem geeigneten solvolytischen Medium bewerkstelligt werden. Als solvolytisches Medium kommen wäßrige Medien in Betracht, die ein mischbares Co-solvens enthalten. Geeignete Co-solventien sind ätherische Lösungsmittel wie Tetrahydrofuran oder Dioxan, oder Ketone wie Aceton und Methyläthylketon oder Alkohole, wie Äthanol. Als Säuren kommen Mineralsäuren wie Salzsäure, Bromwasserstoffsäure oder Schwefelsäure, oder organische Sulfonsäuren wie Benzolsulfonsäure oder p-Toluolsulfonsäure in Betracht. Bei Verwendung dieser Säuren wird gleichzeitig die Ketalgruppe hydrolysiert.

Das so erhaltene 25,26-Dihydroxy-cholesterin kann, z. B. wie in Steroids 24 (1974), Seite 463, beschrieben, in 25,26-Dihydroxy-cholecalciferol übergeführt werden.

Die folgenden Beispiele erläutern die Erfindung.

## Beispiel 1

23 g S-2-Methylbutan-1,2,4-triol-1,2-acetonid (ca. 86%ig) wurden in 100 ml Methylenchlorid gelöst. In dieser Lösung wurden 38 g Triphenylphosphin gelöst und danach wurden bei 0° innerhalb 15 Minuten 23,2 g N-Bromsuccinimid zugegeben. Das Reaktionsgemisch wurde 1 Stunde bei Raumtemperatur gerührt, dann mit 300 ml Hexan versetzt und durch 100 g Kieselgel filtriert. Nach Waschen mit Hexan wurde das Filtrat eingedampft. Man erhielt als Rückstand 20,9 g (4S),4-(2-Bromäthyl)-2,2,4-trimethyl-1,3-dioxolan als farbloses Öl, $[\alpha]_D = +2,89°$ (c=4,1% in Chloroform).

## Beispiel 2

19,2 g (4S),4(2-Bromäthyl)-2,2,4-trimethyl-1,3-dioxolan wurden zu einer Suspension von 2,5 g Magnesiumspänen in 100 ml Tetrahydrofuran gegeben. Das Reaktionsgemisch wurde 1/2 Stunde zum Rückfluß erhitzt, anschließend auf 0° gekühlt und mit 32 g (20S)-6β-Methoxy-20-methyl-3α,5-cyclo-5α-pregnan-21-al versetzt. Das Reaktionsgemisch wurde 3 Stunden bei 0° und 15 Stunden bei Raumtemperatur gerührt, danach mit 50 ml gesättigter Ammoniumchloridlösung versetzt und mit dreimal 100 ml Äther extrahiert. Der ätherische Extrakt wurde über Magnesiumsulfat getrocknet und eingedampft. Der Rückstand wurde an Kieselgel mit Toluol : Äthylacetat (2 : 1) chromatographiert. Man erhielt 20,3 g (22RS)-6β-Methoxy-24[(S)-2,2,4-trimethyl-1,3-dioxolan-4-yl]3α,5-cyclo-5α-cholan-22-ol. $[\alpha]_D + 33,4°$ (c=1,7% in Chloroform).

## Beispiel 3

10 g (22RS)-6β-Methoxy-24[(S)-2,2,4-trimethyl-1,3-dioxolan-4-yl]3α,5-cyclo-5α-cholan-22-ol wurden in 31,4 ml Methansulfonylchlorid und 179 ml Pyridin gelöst. Die Lösung wurde über Nacht stehen gelassen, sodann mit 400 g Eis versetzt und 1 Stunde gerührt. Danach wurde das Reaktionsgemisch filtriert und mit fünfmal 50 ml Wasser gewaschen. Der Feststoff wurde in Methylenchlorid gelöst, die Lösung über Magnesiumsulfat getrocknet und eingedampft. Man erhielt ein Rohprodukt, das an 300 g Kieselgel mit Toluol : Äthylacetat (2 : 1) chromatographiert wurde. Man erhielt 10,3 g (22RS)-6β-Methoxy-24[(S)-2,2,4-trimethyl-1,3-dioxolan-4-yl]3α,5-cyclo-22-mesyloxy-5α-cholan vom Schmelzpunkt 118—120°, $[\alpha]_D = 34,8°$ (c=2,6% in Chloroform).

## Beispiel 4

10,3 g (22RS)-6β-Methoxy-24[(S)-2,2,4-trimethyl-1,3-dioxolan-4-yl]3α,5-cyclo-22-mesyloxy-5α-cholan wurden in 100 ml Tetrahydrofuran gelöst und die Lösung wurde mit 10,3 g Lithiumaluminiumhydrid versetzt. Das Reaktionsgemisch wurde 2 Stunden zum Rückfluß erhitzt, danach auf 0° gekühlt, mit 60 ml Methanol und danach tropfenweise mit 60 ml Wasser versetzt. Das Reaktionsprodukt wurde mit dreimal 100 ml Äther extrahiert, die ätherische Phase über Magnesiumsulfat getrocknet und eingedampft. Der Rückstand wurde in 100 ml Dioxan aufgenommen und in Gegenwart von 4 g Pt-Kohle (10%ig) 1 Stunde unter Wasserstoffatmosphäre geschüttelt. Danach wurde die Lösung filtriert und das Filtrat eingedampft. Man erhielt 7,82 g (22RS)-6β-Methoxy-24[(S)-2,2,4-trimethyl-1,3-dioxolan-4-yl]-3α,5-cyclo-5α-cholan, $[\alpha]_D = 28,4°$ (c=3,8% in Chloroform).

Beispiel 5

7,8 g (22RS)-6β-Methoxy-24[(S)-2,2,4-trimethyl-1,3-dioxolan-4-yl]3α,5-cyclo-5α-cholan wurden in 200 ml Dioxan gelöst und nach Zusatz von 0,3 g p-Toluolsulfonsäure und 60 ml Wasser 3 Stunden zum Rückfluß erhitzt. Danach wurde das Reaktionsgemisch 12 Stunden bei Raumtemperatur gerührt und mit 200 ml Äther versetzt. Die wäßrige Phase wurde mit zweimal 50 ml Äther gewaschen, und die vereinigten organischen Phasen über Magnesiumsulfat getrocknet und eingedampft. Der Rückstand wurde in Äther suspendiert, die Suspension filtriert und der Rückstand mit Äther gewaschen. Nach Trocknen des Rückstandes erhielt man 3,6 g 25S,26-Dihydroxy-cholesterin, $[\alpha]_D$ −35,8° (c=0,4% in Methanol).

Durch Behandlung des so erhaltenen 25S, 26-Dihydroxy-cholesterins mit Acetanhydrid und Pyridin wurde das 25S,26-Dihydroxy-cholesterin-3,26-diacetat, $[\alpha]_D$ −38,6° (c=1,4% in Chloroform) erhalten.

**Patentansprüche**

1. Verfahren zur Herstellung von 25,26-Dihydroxy-cholesterin, dadurch gekennzeichnet, daß man

a) einen i-Steroidaldehyd der Formel

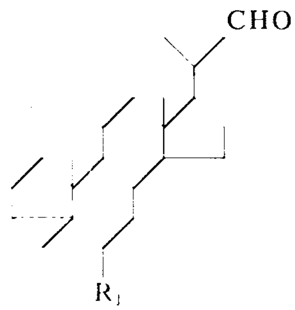

(II)

worin $R_1$ nieder-Alkoxy ist,

mit einem Halogenketal der Formel

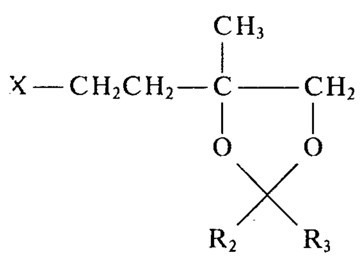

(III)

worin X Chlor, Brom oder Jod ist und $R_2$ und $R_3$ nieder-Alkyl oder $R_2$ und $R_3$ zusammen nieder-Alkylen sind,

in einer Grignard-Reaktion umsetzt,

b) den erhaltenen 22-Steroidalkohol der Formel

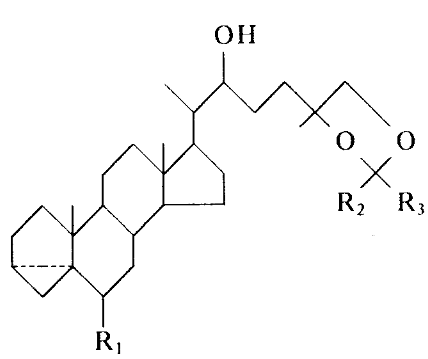

(I)

worin $R_1$, $R_2$ und $R_3$ die obige Bedeutung haben,
mit einer Sulfonsäure verestert,

c) den erhaltenen 22-Sulfonsäureester der Formel

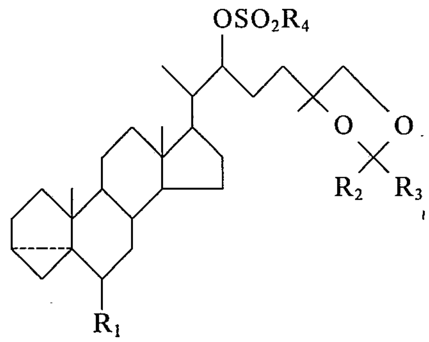

(IV)

worin $R_1$, $R_2$ und $R_3$ die obige Bedeutung haben und $R_4$ nieder-Alkyl, Phenyl oder substituiertes Phenyl ist,
entweder direkt durch Behandlung mit einem komplexen Metallhydrid oder zweistufig durch Abspaltung der Sulfonsäure und unter Ausbildung einer 22,23-Doppelbindung und Hydrierung des erhaltenen Steroidketals der Formel

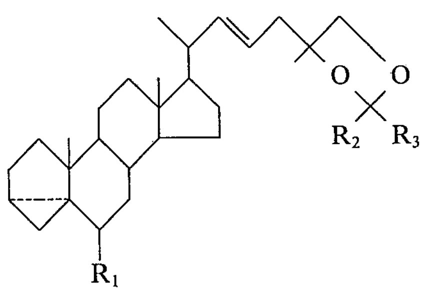

(VI)

worin $R_1$, $R_2$ und $R_3$ die obige Bedeutung haben,
in ein Steroidketal der Formel

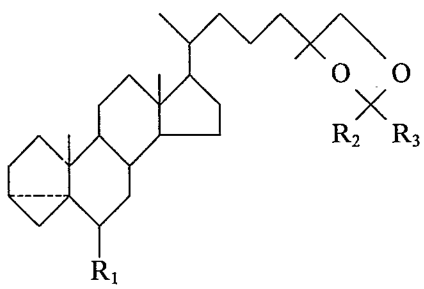

(V)

worin $R_1$, $R_2$ und $R_3$ die obige Bedeutung haben,
überführt,

d) das Steroidketal der Formel V einer retro-i-Umlagerung unterwirft und die Ketalgruppe hydrolysiert.

2. Verfahren nach Anspruch 1, dadurch gekennzeichnet, daß man von einem (20S)-i-Steroidaldehyd ausgeht.

3. Verfahren nach Anspruch 1 oder 2, dadurch gekennzeichnet, daß man den i-Steroidaldehyd mit der Grignard-Verbindung einer optisch aktiven Verbindung der Formel III umsetzt.

4. Cholestanderivate der Formel

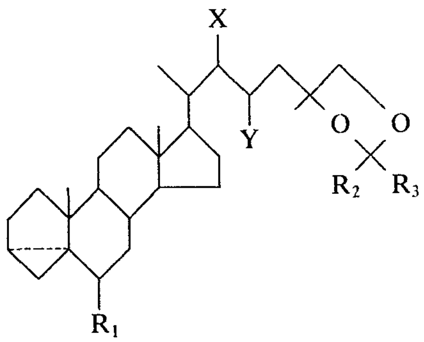

(Ia)

worin $R_1$ nieder-Alkoxy, $R_2$ und $R_3$ nieder-Alkyl oder $R_2$ und $R_3$ zusammen nieder-Alkylen, X Hydroxy, eine Gruppe der Formel $OSO_2R_4$ oder Wasserstoff und Y Wasserstoff oder X und Y zusammen eine C-C-Bindung und $R_4$ nieder-Alkyl, Phenyl oder substituiertes Phenyl sind.

5. Cholestanderivate der Formel Ia nach Anspruch 4, worin X Hydroxy und Y Wasserstoff sind.
6. (22RS)-6β-Methoxy-24[(S)-2,2,4-trimethyl-1,3-dioxolan-4-yl]-3α,5-cyclo-5α-cholan-22-ol.
7. (22RS)-6β-Methoxy-24-[(S)-2,2,4-trimethyl-1,3-dioxolan-4-yl]-3α,5-cyclo-22-mesyloxy-5α-cholan.
8. (22RS)-6β-Methoxy-24-[(S)-2,2,4-trimethyl-1,3-dioxolan-4-yl]-3α,5-cyclo-5α-cholan.

**Claims**

1. Process for the manufacture of 25,26-dihydroxy-cholesterol, characterized by

a) reacting a i-steroid aldehyde of the formula

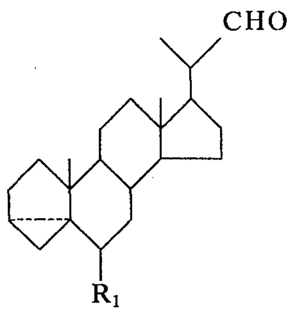

(II)

wherein $R_1$ is lower-alkoxy,
with a haloketal of the formula

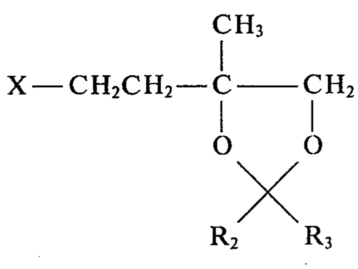

(III)

wherein X is chlorine, bromine or iodine and $R_2$ and $R_3$ are lower-alkyl or $R_2$ and $R_3$ together are lower-alkylene,
in a Grignard reaction,
b) esterifying the resulting 22-steroid alcohol of the formula

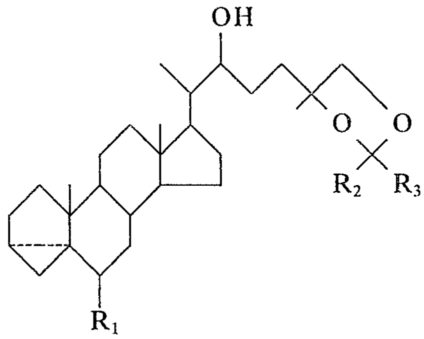

(I)

wherein $R_1$, $R_2$ and $R_3$ have the above significance,

with a sulphonic acid,

c) converting the resulting 22-sulphonic acid ester of the formula

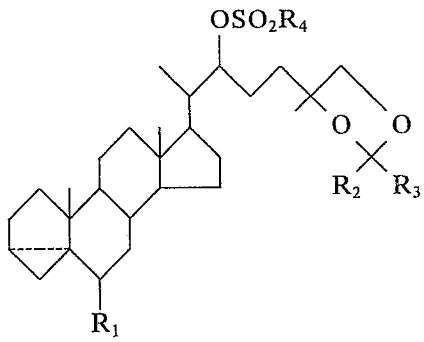

(IV)

wherein $R_1$, $R_2$ and $R_3$ have the above significance and $R_4$ is lower-alkyl, phenyl or substituted phenyl,

either directly by treatment with a complex metal hydride or in two steps by cleavage of the sulphonic acid with formation of a 22,23-double bond and hydrogenation of the resulting steroid ketal of the formula

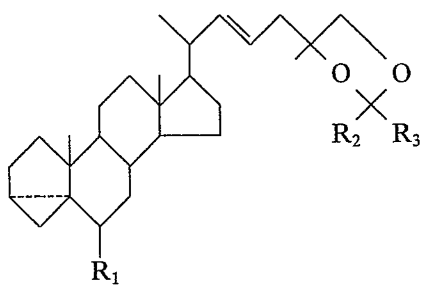

(VI)

wherein $R_1$, $R_2$ and $R_3$ have the above significance,

into a steroid ketal of the formula

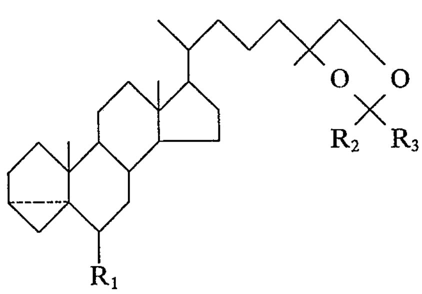

(V)

wherein $R_1$, $R_2$ and $R_3$ have the above significance,

d) subjecting the steroid ketal of formula V to a retro-i-rearrangement and hydrolyzing the ketal group.

2. Process according to claim 1, characterized in that a (20S)-i-steroid aldehyde is used as the starting material.

3. Process according to claim 1 or 2, characterized in that the i-steroid aldehyde is reacted with the Grignard compound of an optically active compound of formula III.

4. Cholestane derivatives of the formula

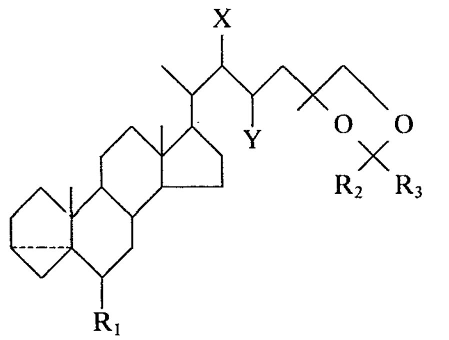

(Ia)

wherein $R_1$ is lower-alkoxy, $R_2$ and $R_3$ are lower-alkyl or $R_2$ and $R_3$ together are lower-alkylene, X is hydroxy, a group of the formula $OSO_2R_4$ or hydrogen and Y is hydrogen or X and Y together are a C-C bond and $R_4$ is lower-alkyl, phenyl or substituted phenyl.

5. Cholestane derivatives of formula Ia according to claim 4, wherein X is hydroxy and Y is hydrogen.

6. (22RS)-6β-Methoxy-24[(S)-2,2,4-trimethyl-1,3-dioxolan-4-yl]-3a,5-cyclo-5α-cholan-22-ol.

7. (22RS)-6β-Methoxy-24-[(S)-2,2,4-trimethyl-1,3-dioxolan-4-yl]-3α,5-cyclo-22-mesyloxy-5α-cholane.

8. (22RS)-6β-Methoxy-24-[(S)-2,2,4-trimethyl-1,3-dioxolan-4-yl]-3α,5-cyclo-5α-cholane.

**Revendications**

1. Procédé de préparation du 25,26-dihydroxycholestérol, caractérisé en ce que

a) on fait réagir dans une réaction de Grignard un aldéhyde istéroîdique de formule

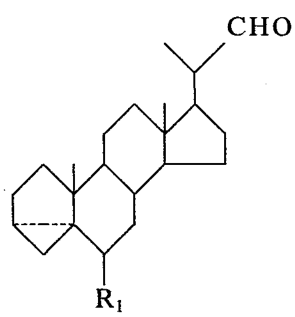

(II)

dans laquelle $R_1$ représente un groupe alcoxy,

avec un halogénoacétal de formule

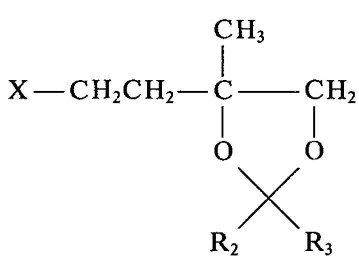

(III)

dans laquelle X représente le chlore, le brome ou l'iode et $R_2$ et $R_3$ représentent des groupes alkyles inférieurs

ou forment ensemble un groupe alkylène inférieur,

b) on estérifie le 22-alcool stéroîdique obtenu, de formule

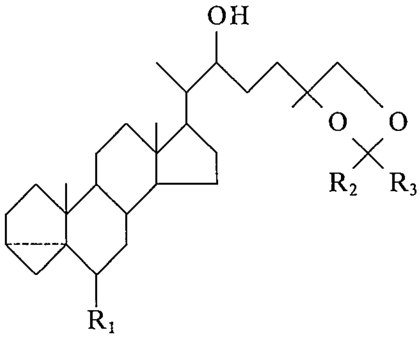

(I)

dans laquelle $R_1$, $R_2$ et $R_3$ ont les significations indiquées ci-dessus, par un acide sulfonique,

c) on convertit le 22-ester d'acide sulfonique obtenu, de formule

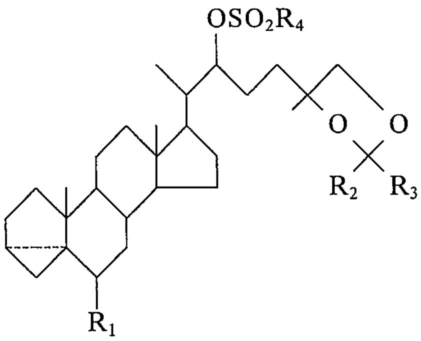

(IV)

dans laquelle $R_1$, $R_2$ et $R_3$ ont les significations ci-dessus et $R_4$ représente un groupe alkyle inférieur, phényle ou phényle substitué,

soit directement par traitement à l'aide d'un hydrure métallique complexe, soit en deux stades opératoires par scission de l'acide sulfonique et avec formation d'une double liaison en 22, 23 et hydrogénation de l'acétal stéroïdique obtenu, de formule

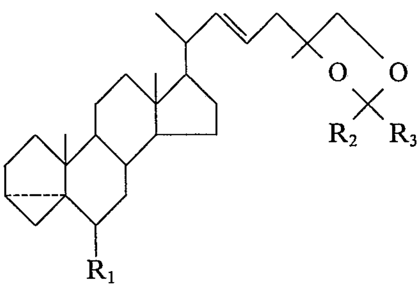

(VI)

dans laquelle $R_1$, $R_2$ et $R_3$ ont les significations indiquées ci-dessus, en un acétal stéroïdique de formule

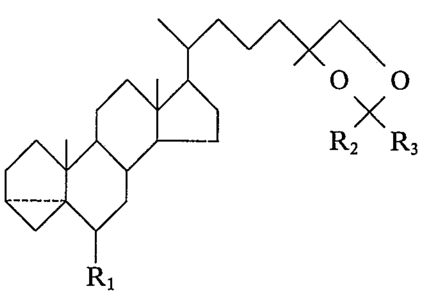

(V)

dans laquelle $R_1$, $R_2$ et $R_3$ ont les significations indiquées ci-dessus,

d) on soumet l'acétal stéroïdique de formule V à une rétro-i-transposition et on hydrolyse le groupe acétal.

2. Procédé selon la revendication 1, caractérisé en ce que l'on part d'un (20S)-aldéhyde i-stéroïdique.

3. Procédé selon la revendication 1 ou 2, caractérisé en ce que l'on fait réagir l'aldéhyde i-stéroïdique avec le composé de Grignard d'un énantiomère de formule III.

4. Dérivés du cholestane de formule

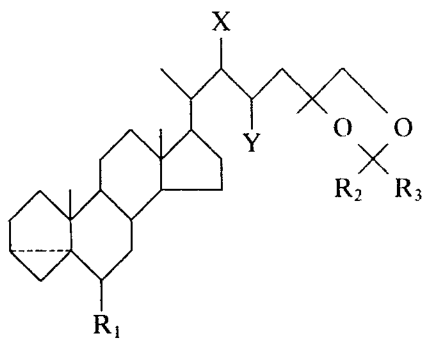

(Ia)

dans laquelle $R_1$ représente un groupe alcoxy inférieur, $R_2$ et $R_3$ représentent des groupes alkyles inférieurs ou bien $R_2$ et $R_3$ forment ensemble un groupe alkylène inférieur, X représente un groupe hydroxy, un groupe de formule $OSO_2R_4$ ou l'hydrogène et Y représente l'hydrogène ou bien X et Y forment ensemble une liaison C-C et $R_4$ représente un groupe alkyle inférieur, phényle ou phényle substitué.

5. Dérivés du cholestane de formule Ia selon la revendication 4 dans laquelle X représente un groupe hydroxy et Y l'hydrogène.

6. Le (22RS)-6bêta-méthoxy-24-[(S)-2,2,4-triméthyl-1,3-dioxolanne-4-yl]-3alpha, 5-cyclo-5alpha-cholane-22-ol.

7. Le (22RS)-6bêta-méthoxy-24-[(S)-2,2,4-triméthyl-1,3-dioxolanne-4-yl]-3alpha, 5-cyclo-22-mésyloxy-5alpha-cholane.

8. Le (22RS)-6bêta-méthoxy-24-[(S)-2,2,4-triméthyl-1,3-dioxolanne-4-yl]-3alpha, 5-cyclo-5alpha-cholane.